# EUROPEAN PATENT SPECIFICATION

(11) **EP 1 084 107 B1**
(45) Date of publication and mention of the grant of the patent: **11.04.2007**
(21) Application number: 98961866.5
(22) Date of filing: 03.12.1998
(51) Int. Cl.: C07D 207/16, A61K 31/40, C07D 207/12, C07D 403/04, A61K 31/41

(54) **AZA-HETEROCYCLIC COMPOUNDS USED TO TREAT NEUROLOGICAL DISORDERS AND HAIR LOSS**
AZAHETEROZYKLISCHEDERIVATE ZUR BEHANDLUNG VON HAARAUSFALL UND NEUROLOGISCHER KRANKHEITEN
COMPOSES AZA-HETEROCYCLIQUES UTILISES POUR TRAITER LES TROUBLES NEUROLOGIQUES ET LA PERTE DES CHEVEUX

(30) Priority: 03.06.1998 US 87788 P; 18.09.1998 US 101077 P
(43) Date of publication of application: 21.03.2001
(73) Proprietor: GPI NIL Holdings, Inc., Baltimore, MD 21224 (US); Amgen Inc.,, Thousand Oaks, California 91320-1799 (US)
(72) Inventor: HAMILTON, Gregory, S., Catonsville, MD 21228 (US); NORMAN, Mark, H., Thousand Oaks, CA 91360 (US); WU, Yong-Qian, Columbia, MD 21044 (US)
(74) Representative: HOFFMANN EITLE
(86) International application number: PCT/US1998/025573
(87) International publication number: WO 1999/062881

(56) References cited:
- WO-A-92/00278
- WO-A-96/06097
- WO-A-96/40633
- WO-A-97/31898
- WO-A-98/37885
- WO-A-98/55090
- WO-A-98/55091

## Description

### Related Application Data

This application is a continuation-in-part of U.S. patent application serial number 60/087, 788 to Hamilton et al., entitled "Carboxylic Acids and Carboxylic Acid Isosteres of N-Heterocyclic Compounds", filed June 3, 1998, of U.S. patent application serial number 60/101, 077 to Hamilton et al., entitled "Carboxylic Acids and Carboxylic Acid Isosteres of N-Heterocyclic Compounds", filed September 18, 1998.

### BACKGROUND OF THE INVENTION

### 1. Field of the Invention

This invention relates to novel carboxylic acid isosteres of N-hezerocylic compounds, their preparation and use for treating neurological disorders including physically damaged nerves and neurodegenerative diseases, and for treating alopecia and promoting hair growth.

### 2. Description of the Prior Art

It has been found that picomolar concentrations of an immunosuppressant such as FK506 and rapamycin stimulate neurite out growth in PC12 cells and sensory nervous, namely dorsal root ganglion cells (DRGs). Lyons et al., *Proc. of Natl. Acad. Sci.,* 1994 vol. 91, pp. 3191-3195. In whole animal experiments, FK506 has been shown to stimulate nerve regeneration following facial nerve injury and results in functional recovery in animals with sciatic nerve lesions.

Several neurotrophic factors effecting specific neuronal populations in the central nervous system have been identified. For example, it has been hypothesized that Alzheimer's disease results from a decrease or loss of nerve growth factor (NGF). It has thus been proposed to treat Alzheimer's patients with exogenous nerve growth factor or other neurotrophic proteins such as brain derived nerve factor (BDNF), glial derived nerve factor, ciliary neurotrophic factor, and neurotropin-3 to increase the survival of degenerating neuronal populations.

Clinical application of these proteins in various neurological disease states is hampered by difficulties in the delivery and bioavailability of large proteins to nervous system targets. By contrast, immunosuppressant drugs with neurotrophic activity are relatively small and display excellent bioavailability and specificity. However, when administered chronically, immunosuppressants exhibit a number of potentially serious side effects including nephrotoxicity, such as impairment of glomerular filtration and irreversible interstitial fibrosis (Kopp et al., 1991, J. Am. Soc. Nephrol. 1:162); neurological deficits, such as involuntary tremors, or non-specific cerebral angina such as non-localized headaches (De Groen et al., 1987, N. Engl. J. Med. 317:861); and vascular hypertension with complications resulting therefrom (Kahan et al., 1989 N. Engl. J. Med. 321: 1725).

Accordingly, there is a need for small-molecule compounds which are useful for neurotrophic effects and for treating neurodegenerative disorders.

Hair loss occurs in a variety of situations. These situations include male pattern alopecia, alopecia senilis, alopecia areata, diseases accompanied by basic skin lesions or tumors, and systematic disorders such as nutritional disorders and internal secretion disorders. The mechanisms causing hair loss are very complicated, but in some instances can be attributed to aging, genetic disposition, the activation of male hormones, the loss of blood supply to hair follicles, and scalp abnormalities.

The immunosuppressant drugs FK506, rapamycin and cyclosporin are well known as potent T-cell specific immunosuppressants, and are effective against graft rejection after organ transplantation. It has been reported that topical, but not oral, application of FK506 (Yamamoto et al., J. Invest. Dermatol., 1994, 102, 160-164; Jiang et al., J. Invest. Dermatol. 1995, 104, 523-525) and cyclosporin (Iwabuchi et al., J. Dermatol. Sci. 1995, 9, 64-69) stimulates hair growth in a dose-dependent manner. One form of hair loss, alopecia areata, is known to be associated with autoimmune activities; hence, topically administered immumodulatory compounds are expected to demonstrate efficacy for treating that type of hair loss. The hair growth stimulating effects of FK506 have been the subject of an international parent filing covering FK506 and structures related thereto for hair growth stimulation (Honbo et al., EP 0 423 714 A2). Honbo et al. discloses the use of relatively large tricyclic compounds, known for their immunosuppressive effects, as hair revitalizing agents.

The hair growth and revitalization effects of FK506 and related agents are disclosed in many U.S. patents (Goulet et al., U.S. Patent No. 5, 258, 389; Luly et al., U.S. Patent No. 5, 457, 111; Goulet et al., U.S. Patent No. 5,532,248; Goulet et al., U.S. Patent No. 5, 189, 042; and Ok et al., U.S. Patent No. 5,208,241; Rupprecht et al., U.S. Patent No. 5,284,840; Organ et al., U.S. Patent No. 5,284,877). These patents claim FK506 related compounds. Although they do not claim methods of hair revitalization, they disclose the known use of FK506 for affecting hair growth. Similar to FK506 (and the claimed variations in the Honbo et al. patent), the compounds claimed in these patents are relatively large. Further, the cited patents relate to immunomodulatory compounds for use in autoimmune related diseases, for which FK506's efficacy is well known.

Other U.S. patents disclose the use of cyclosporin and related compounds for hair revitalization (Hauer et al., U.S. Patent No. 5, 342, 625; Eberle, U.S. Patent No. 5,284,826; Hewitt et al., U.S. Patent No. 4,996,193). These patents also relate to compounds useful for treating autoimmune diseases and cite the known use of cyclosporin and related immunosuppressive compounds for hair growth.

However, immunosuppressive compounds by definition suppress the immune system and also exhibit other toxic side effects. Accordingly, there is a need for small molecule compounds which are useful as hair revitalizing compounds. J.S.C. Chem. Comm. 1975, 24, 988-989 discloses the compounds (2S)-1-(1,2-dioxo-2-methyl)-2-pyrrolidinecarboxamide and (2S)-1-(1,2-dioxo-2-methyl)-2-pyrrolidinecarbonitrile as synthetic intermediates for the synthesis of α-amino acids. EP-A-0 048 159 discloses the compounds (2S)-1-(1,2-dioxo-2-isopropyl)-2-pyrrolidinecarboxamide, (2S)-1-(1,2-dioxo-2-isobutyl)-2-pyrrolidinecarboxamide, (2S)-1-(1,2-dioxo-2-isopentyl)-2-pyrrolidinecarboxamide, and (2S)-1-[1,2-dioxo-2-(4-methylpentyl)]-2-pyrrolidinecarboxamide as intermediates for the preparation of ACE-inhibitors.

### SUMMARY OF THE INVENTION

The present invention relates to the surprising discovery that N-heterocyclic compounds containing a carboxylic acid isostere moiety may be useful for treating neurodegenerative disorders and for treating alopecia and related hair loss disorders. Accordingly, a novel class of compounds containing an acidic moiety or an isostere thereof attached to the 2-carbon of the N-heterocyclic ring is provided. These compounds stimulate neuronal regeneration and outgrowth and as such are useful for treating neurological disorders and neurodegenerative diseases. These compounds also promote hair growth and as such are useful for treating hair loss disorders. A preferred feature of the compounds of the present invention is that they do not exert any significant immunosuppressive activity and/or are non-immunosuppressive.

More specifically, this invention relates to a compound of formula (I): 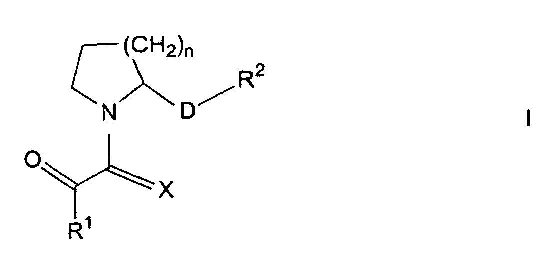 or a pharmaceutically acceptable salt, ester, or solvate of the compound, wherein:
n is 1-3;
X is either 0 or S;
R¹ is C₁-C₉ straight or branched chain alkyl, or C₂-C₉ straight or branched chain alkenyl,
D is a bond, C₁-C₁₀ straight or branched chain alkylene, C₂-C₁₀ alkenylene, or C₂-C₁₀ alkynylene; and
R² is tetrazolyl, -CONH₂, -CN, -SO₃H or -OH;
wherein said alkyl, alkenyl, alkenylene and alkynylene are optionally substituted with one or more substituents independently selected from hydroxy, halo, C₁-C₆ haloalkyl, thiocarbonyl, C₁-C₆ alkoxy, C₂-C₆ alkenoxy, cyano, nitro, imino, C₁-C₆ alkylamino, C₁-C₆ aminoalkyl, sulfhydryl, C₁-C₆ thioalkyl, C₁-C₆ alkylthio, sulfonyl, C₁-C₆ straight or branched chain alkyl, C₂-C₆ straight or branched chain alkenyl or alkynyl, or CO₂R⁷ where R⁷ is hydrogen, C₁-C₉ straight or branched chain alkyl or C₂-C₉ straight or branched chain alkenyl;
**provided that:**
when n=1, X is O, D is a bond, and R² is -CONH₂,
**then** R¹ is not methyl, ethyl, iso-propyl, iso-butyl, iso-pentyl, or 4-methylpentyl; and
**further provided that:**
when n=1, X is O, D is a bond, and R² is cyano,
**then** R₁ is not methyl.

An especially preferred embodiment of this invention is where R₂ is

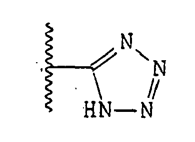

Another preferred embodiment of this invention is where R₂ is selected from the group consisting of -SO₃H, and -CN.

Preferred embodiments of this invention are: (2S)-1-(1,2-dioxo-3,3-dimethylpentyl)-2-hydroxymethyl pyrrolidine; (2S)-1-(1,2-dioxo-3,3-dimethylpentyl)-2-pyrrolidinetetrazole; (2S)-1-(1,2-dioxo-3,3-dimethylpentyl)-2-pyrrolidinecarbonitrile; and (2S)-1-(1,2-dioxo-3,3-dimethylpentyl)-2-aminocarbonyl piperidine.

Another preferred embodiment of this invention is a pharmaceutical composition containing: an effective amount of a compound of formula (1); and a pharmaceutically suitable or acceptable carrier. For neurotrophic compositions a neurotrophic factor different from formula (I) may also be administered or otherwise included in the composition.

Another preferred embodiment of the invention is the use of said compounds for the preparation of a medicament for promoting neuronal regeneration and growth in mammals,
for treating a neurological disorder in an animal, to stimulate growth of damaged peripheral nerves or to promote neuronal regeneration.

Yet another preferred embodiment of the invention is the use of said compounds for the preparation of a medicament for preventing neurodegeneration in an animal,
for treating alopecia or for promoting hair growth in an animal.

### BRIEF DESCRIPTION OF THE DRAWINGS

Figure 1 is a photograph of C57 Black 6 mice before being shaved for the hair regeneration experiment.
Figure 2 is a photograph of mice treated with a vehicle after six weeks. Figure 2 shows that less than 3% of the shaved area is covered with new hair growth when the vehicle (control) is administered.
Figure 3 is a bar graph illustrating relative hair growth on shaved mice treated with N-hererocyclic carboxylic acids or carboxylic acid isosteres at 1µmole per milliliter three times per week. Hair growth was evaluated after 14 days of treatment.

### DETAILED DESCRIPTION OF THE INVENTION

### Definitions

"Alkyl" means a branched or unbranched saturated hydrocarbon chain comprising a designated number of carbon atoms. For example, C₁-C₆ straight or branched alkyl hydrocarbon chain contains 1 to 6 carbon atoms, and includes but is not limited to substituents such as methyl, ethyl, propyl, iso-propyl, butyl, iso-butyl, tert-butyl, n-pentyl, n-hexyl, and the like. It is also contemplated as within the scope of the present invention that "alkyl" may also refer to a hydrocarbon chain wherein any of the carbon atoms of said alkyl are optionally replaced with O, NH, S, or SO₂. For example, carbon 2 of n-pentyl can be replaced with O to form propyloxymethyl.

"Alkenyl" means a branched or unbranched unsaturated hydrocarbon chain comprising a designated number of carbon atoms. For example, C₂-C₆ straight or branched alkenyl hydrocarbon chain contains 2 to 6 carbon atoms having at least one double bond, and includes but is nor limited to substituents such as ethenyl, propenyl, iso-propenyl, butenyl, iso-butenyl, tert-butenyl, n-pentenyl, n-hexenyl, and the like. It is also contemplated as within the scope of the present invention that "alkenyl" may also refer to an unsaturated hydrocarbon chain wherein any of the carbon atoms of said alkenyl are optionally replaced with O, NH, S, or SO₂. For example, carbon 2 of 4-pentene can be replaced with O to form (2-propene)oxymethyl.

"Alkoxy" means the group -OR wherein R is alkyl as herein defined. Preferably, R is a branched or unbranched saturated hydrocarbon chain containing 1 to 6 carbon atoms.

Specifically, the term "carbocycle" or refers to an organic cyclic moiety in which the cyclic skeleton is comprised of only carbon atoms whereas the term "heterocycle" refers to an organic cyclic moiety in which the cyclic skeleton contains one or more heteroatoms selected from nitrogen, oxygen, or sulfur and which may or may not include carbon atoms.

Thus, the term "carbocycle" refers to a carbocyclic moiety containing the indicated number of carbon atoms. The term "C₃-C₈ cycloalkyl", therefore, refers to an organic cyclic substituent in which three to eight carbon atoms form a three, four, five, six, seven, or eight-membered ring, including, for example, a cyclopropyl, cyclobuty;, cyclopentyl, cyclohexyl, cycloheptyl, or cyclooctyl ring. As used herein, "carbocycle" may also refer to two or more cyclic ring systems which are fused to form, for example bicyclic, tricyclic, or other similar bridged substituents (e.g. adamatyl).

"Aryl" refers to an aromatic carbocyclic group having a single ring, for example a phenyl ring; multiple rings, for example biphenyl; or multiple condensed rings in which at least one ring is aromatic, for example naphthyl, 1,2,3,4-tetrahydronaphthyl, anthryl, or phenanthryl, which can be unsubstituted or substituted with one or more other substituents as defined above. The substituents attached to a phenyl ring portion of an aryl moiety in the compounds of Formula (I) may be configured in the ortho-, meta-, or para- orientations.

Examples of typical aryl moieties included in the scope of the present invention may include, but are not limited to, the following:

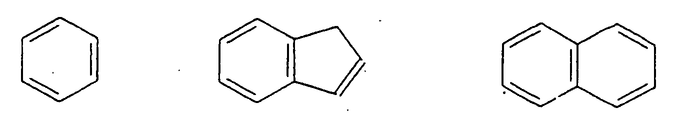

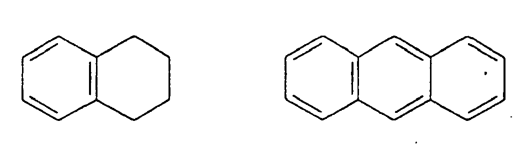

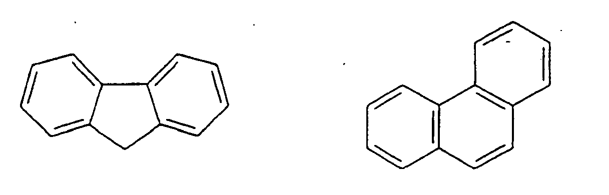

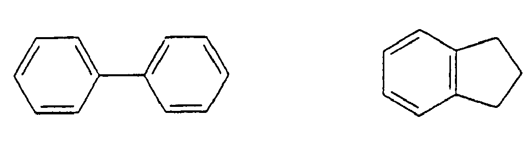

"Aralkyl" refers to alkyl or alkylene (alkenyl) chain which is substituted with aryl, heteroaryl, carbocycle or heterocycle, or alternatively one or more aryl, heteroaryl, carbocycle, or heterocycle(s) which is/are substituted with alkyl or alkenyl, i.e. 'Alkyl/alkylene which is substituted with Ar' or 'Ar which is substituted with alkyl/alkylene'.

"Heterocycle" refers to a saturated, unsaturated, or aromatic carbocyclic group having a single ring, multiple rings, or multiple condensed rings, and having at least one hetero atom such.as nitrogen, oxygen, or sulfur within at least one of the rings. "Heteroaryl" refers to a heterocycle in which at least one ring is aromatic. Any of the heterocyclic or heteroaryl groups can be unsubstituted or optionally substituted with one or more groups as defined above. Further, bi- or tri-cyclic heteroaryl moieties may comprise at least one ring which is either completely or partially saturated.

As one skilled in the art will appreciate, such heterocyclic moieties may exist in several isomeric forms, all of which are encompassed by the present invention. For example, a 1,3,5-triazine moiety is isomeric to a 1,2,4-triazine group. Such positional isomers are to be considered within the scope of the present invention. Likewise, the heterocyclic or heteroaryl groups can be bonded to other moieties in the compounds of the present invention. The point(s) of attachment to these other moieties is not to be construed as limiting on the scope of the invention. Thus, by way of example, a pyridyl moiety may be bound to other groups through the 2-, 3-, or 4-position of the pyridyl group. All such configurations are to be construed as within the scope of the present invention.

Examples of heterocyclic or heteroaryl moieties included in the scope of the present invention may include, but are not limited to, the following:

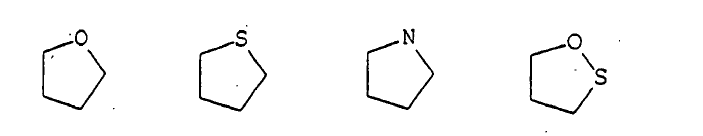

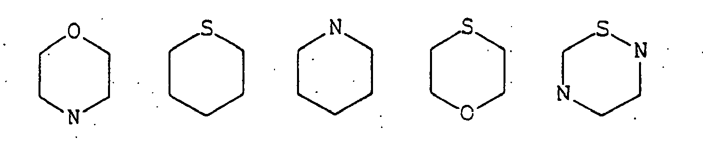

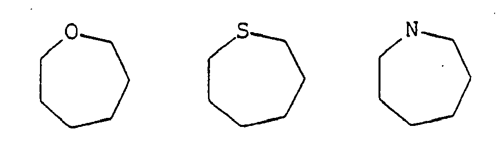

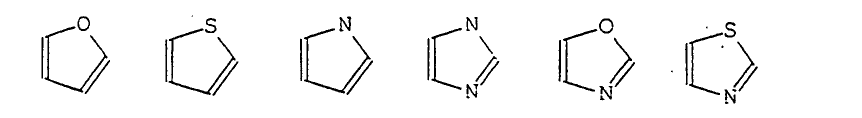

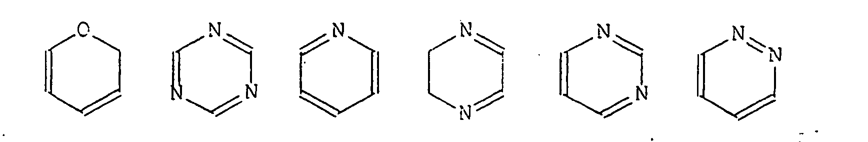

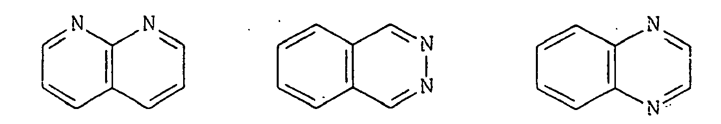

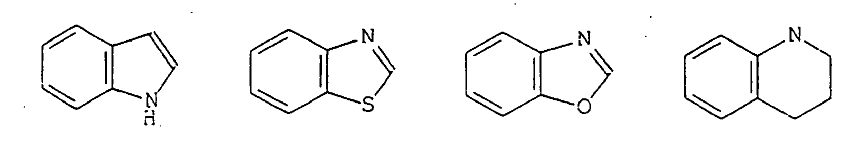

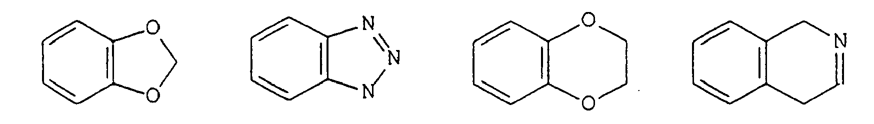

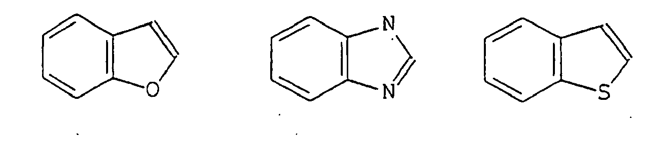

"Halo" means at least one fluoro, chloro, bromo, or iodo moiety.

The term "pharmaceutically acceptable salt, ester, or solvate" refers to salt, ester, or solvates of the subject compounds which possess the desired pharmacological activity and which are neither biologically nor otherwise undesirable. The salt, ester, or solvates can be formed with inorganic or organic acids such as acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, gluconate, glycerophosphate, hemisulfate, heptanoate, hexanoate, hydrochloride hydrobromide, hydroiodide, 2-hydroxyethanesulfonate, lactate, maleate, methanesulfonate, naphthylate, 2-naphchalenesulfonate, nicotinate, oxalate, sulfate, thiocyanate, tosylate and undecanoate. Base salt, ester, or solvates include ammonium salts, alkali metal salts such as lithium, sodium and potassium salts, alkaline earth metal salts such as calcium and magnesium salts, salt with organic bases such as dicyclohexylamine salts, N-methyl-D-glucamine, and salts with amino acids such as arginine, lysine, and so forth. Also, the basic nitrogen-containing groups can be quarternized with such agents as: 1) lower alkyl halides, such as methyl, ethyl, propyl, and butyl chloride, bromides and iodides; 2) dialkyl sulfates like dimethyl, diethyl, dibutyl and diamyl sulfates; 3) long chain alkyls such as decyl, lauryl, myristyl and stearyl substituted with one or more halide such as chloride, bromide and iodide; and 4) aralkyl halides like benzyl and phenethyl bromide and others.

The compounds of this invention may possess at least one asymmetric center and thus can be produced as mixtures of stereoisomers or as individual enantiomers or diastereomers. The individual stereoisomers may be obtained by using an optically active starting material, by resolving a racemic or non-racemic mixture of an intermediate at some appropriate stage of the synthesis, or by resolution of the compound of formula (I). It is understood that the individual stereoisomers as well as mixtures (racemic and non-racemic) of stereoisomers are encompassed by the scope of the present invention. The S-stereoisomer at atom 1 of formula I is a most preferred embodiment of the invention.

"Stereoisomers" are isomers that differ only in the way the atoms are arranged in space.

"Isomers" are different compounds that have the same molecular formula and includes cyclic isomers such as (iso)indole and other isomeric forms of cyclic moieties.

"Enantiomers" are a pair of stereoisomers that are non-superimposable mirror images of each other.

"Diastereoisomers" are stereoisomers which are not mirror images of each other.

"Racemic mixture" means a mixture containing equal parts of individual enantiomers. "Non-racemic mixture" is a mixture containing unequal parts of individual enantiomers or stereoisomers.

"Isosteres" are different compounds that have different molecular formulae but exhibit the same or similar properties. For example, tetrazole is an isostere of carboxylic acid because it mimics the properties of carboxylic acid even though they both have very different molecular formulae. Tetrazole is one of many possible isosteric replacements for carboxylic acid. Other carboxylic acid isosteres contemplated by the present invention include -SO₃H, and -CN.

It is understood that where chemical substitution is indicated then the chemical substituent chosen would form a sufficiently stable compound.

The term "preventing neurodegeneration" as used herein includes the ability to inhibit or prevent neurodegeneration in patients newly diagnosed as having a neurodegenerative disease, or at risk of developing a new degenerative disease and for inhibiting or preventing further neurodegeneration in patients who are already suffering from or have symptoms of a neurodegenerative disease when the compounds are given concurrently.

The term "treatment" as used herein covers any treatment of a disease and/or condition in an animal, particularly a human, and includes:
(i) preventing a disease and/or condition from occurring in a subject which may be predisposed to the disease and/or condition but has not yet been diagnosed as having it;
(ii) inhibiting the disease and/or condition, i.e., arresting its development; or
(iii) relieving the disease and/or condition, i.e., causing regression of the disease and/or condition.

The system used in naming the compounds of the present invention is shown below, using a compound of formula I as an example.

A compound of the present invention, especially formula I, wherein n is 1, X is O, D is a bond, R₁ is 1,1,dimethylpropyl, and R₂ is -CN, is named (2S)-1-(1,2-dioxo-3,3-dimethylpentyl)-2-pyrrolidinecarbonitrile.

"Alopecia" refers to deficient hair growth and partial or complete loss of hair, including without limitation androgenic alopecia (male pattern baldness), toxic alopecia, alopecia senilis, alopecia areata, alopecia pelada and trichotillomania. Alopecia results when the pilar cycle is disturbed. The most frequent phenomenon is a shortening of the hair growth or anagen phase due to cessation of cell proliferation. This results in an early onset of the catagen phase, and consequently a large number of hairs in the telogen phase during which the follicles are detached from the dermal papillae, and the hairs fall out. Alopecia has a number of etiologies, including genetic factors, aging, local and systemic diseases, febrile conditions, mental stresses, hormonal problems, and secondary effects of drugs.

"Pilar cycle" refers to the life cycle of hair follicles, and includes three phases:
(1) the anagen phase, the period of active hair growth which, insofar as scalp hair is concerned, lasts about three to five years;
(2) the catagen phase, the period when growth stops and the follicle atrophies which, insofar as scalp hair is concerned, lasts about one to two weeks; and
(3) the telogen phase, the rest period when hair progressively separates and finally falls out which; insofar as scalp hair is concerned, lasts about three to four months.
Normally 80 to 90 percent of the follicles are in the anagen phase, less than 1 percent being in the catagen phase, and the rest being in the telogen phase. In the telogen phase, hair is uniform in diameter with a slightly bulbous, non-pigmented root. By contrast, in the anagen phase, hair has a large colored bulb at its root.

"Promoting hair growth" refers to maintaining, inducing, stimulating, accelerating, or revitalizing the germination of hair.

"Treating alopecia" refers to:
(i) preventing alopecia in an animal which may be predisposed to alopecia; and/or
(ii) inhibiting, retarding or reducing alopecia; and/or
(iii) promoting hair growth; and/or
(iv) prolonging the anagen phase of the hair cycle; and/or
(v) converting vellus hair to growth as terminal hair. Terminal hair is coarse, pigmented, long hair in which the bulb of the hair follicle is seated deep in the dermis. Vellus hair, on the other hand, is fine, thin, non-pigmented short hair in which the hair bulb is located superficially in the dermis. As alopecia progresses, the hairs change from the terminal to the vellus type.

The term "neurotrophic" as used herein includes without limitation the ability to stimulate neuronal regeneration or growth and/or the ability to prevent or treat neurodegeneration.

The term "non-immunosuppressive" refers to the inability of the compounds of the present invention to trigger an immune response when compared to a control such as FK506 ro cyclosporin A. Assays for determining immunosuppression are well known to those of ordinary skill in the art. Specific non-limiting examples of well known assays include PMA and OKT3 assays wherein mitogens are used to stimulate proliferation of human peripheral blood lymphocytes (PBC). Compounds added to such assay systems are evaluated for their ability to inhibit such proliferation.

### Compounds of the Invention

The present invention relates to the surprising discovery that carboxylic acid isostere compounds are neurotrophic and are able to treat alopecia. Accordingly, a novel class of compounds are provided. A preferred feature of the compounds of the present invention is that they do not exert any significant immunosuppressive activity.

Preferred compounds of the present invention contain isosteric replacements for carboxylic acid moieties, of which several examples are specified herein. Other isosteric replacements for carboxylic acid moieties, known to those skilled in the art of medicinal chemistry, are within the scope of the invention if not otherwise specified.

The compounds of this invention can be periodically administered to a patient undergoing treatment for neurological disorders or for other reasons in which it is desirable to stimulate neuronal regeneration and growth, such as in various peripheral neuropathic and neurological disorders relating to neurodegeneration. The compounds of this invention can also be administered to mammals other than humans for treatment of various mammalian neurological disorders.

The novel compounds of the present invention possess an excellent degree of neurotrophic activity. This activity is useful in the stimulation of damaged neurons, the promotion of neuronal regeneration, the prevention of neurodegeneration, and in the treatment of several neurological disorders known to be associated with neuronal degeneration and peripheral neuropathies. The neurological disorders that may be treated include but are not limited to: trigeminal neuralgia, glossopharyngeal neuralgia, Bell's Palsy, myasthenia gravis, muscular dystrophy, amyotrophic lateral sclerosis, progressive muscular atrophy, progressive bulbar inherited muscular atrophy, herniated, ruptured or prolapsed invertebrate disk syndromes, cervical spondylosis, plexus disorders, thoracic outlet destruction syndromes, peripheral neuropathic such as those caused by lead, dapsone, ticks, prophyria, or Gullain-Barre syndrome, Alzheimer's disease, and Parkinson's disease.

The above discussion relating to the utility and administration of the compounds of the present invention also applies to the pharmaceutical compositions of the present invention.

The term "pharmaceutically acceptable carrier" as used herein refers to any carrier, diluent, excipient, suspending agent, lubricating agent, adjuvant, vehicle, delivery system, emulsifier, disintegrant, absorbant, preservative, surfactant, colorant, flavorant, or sweetener.

For these purposes the compounds of the present invention may be administered orally, parenterally, by inhalation spray, topically, rectally, nasally, buccally, vaginally or via an implanted reservoir in dosage formulations containing conventional non-toxic pharmaceutically-acceptable carriers, adjuvants and vehicles. The term parenteral as used herein includes subcutaneous, intravenous, intramuscular, intraperitoneally, intrathecally, intraventricularly, intrasternal and intracranial injection or infusion techniques.

For oral administration, the compounds of the present invention may be provided in any suitable dosage form known in the art. For example, the compositions may be incorporated into tablets, powders, granules, beads, chewable lozenges, capsules, liquids, aqueous suspensions or solutions, or similar dosage forms, using conventional equipment and techniques known in the art. Tablet dosage forms are preferred. Tablets may contain carriers such as lactose and corn starch, and/or lubricating agents such as magnesium stearate. Capsules may contain diluents including lactose and dried corn starch. Aqueous suspensions may contain emulsifying and suspending agents combined with the active ingredient.

When preparing dosage form incorporating the compositions of the invention, the compounds may also be blended with conventional excipients such as binders, including gelatin, pregelatinized starch, and the like; lubricants, such as hydrogenated vegetable oil, stearic acid, and the like; diluents, such as lactose, mannose, and sucrose; disintegrants, such as carboxymethylcellulose and sodium starch glycolate; suspending agents, such as povidone, polyvinyl alcohol, and the like; absorbents, such as silicon dioxide; preservatives, such as methylparaben, propylparaben, and sodium benzoate; surfactants, such as sodium lauryl sulfate, polysorbate. 80, and the like; colorants such as F.D.& C. dyes and lakes; flavorants; and sweeteners.

Compositions and methods of the invention also may utilize controlled release technology. Thus, for example, the inventive compounds may be incorporated into a hydrophobic polymer matrix for controlled release over a period of days. Such controlled release films are well known to the art. Particularly preferred are transdermal delivery systems. Other examples Polymers commonly employed for this purpose that may be used in the present invention include nondegradable ethylene-vinyl acetate copolymer and degradable lactic acid-glycolic acid copolymers which may be used externally or internally. Certain hydrogels such as poly(hydroxyethylmethacrylate) or poly(vinylalcohol) also may be useful, but for shorter release cycles then the other polymer releases systems, such as those mentioned above.

To be effective therapeutically as central nervous system targets, the compounds of the present invention should readily penetrate the blood-brain barrier when peripherally administered. Compounds which cannot penetrate the blood-brain barrier can be effectively administered by an intraventricular route or other appropriate delivery system suitable for administration to the brain.

The compounds of the present invention may be administered in the form of sterile injectable preparations, for example, as sterile injectable aqueous or oleaginous suspensions. These suspensions may be formulated according to techniques known in the art using suitable dispersing or wetting agents and suspending agents. The sterile injectable preparations may also be sterile injectable solutions or suspensions in non-toxic parenterally-acceptable diluents or solvents, for example, as solutions in 1,3-butanediol. Among the acceptable vehicles and solvents that may be employed are water, Ringer's solution and isotonic sodium chloride solution. In addition, sterile, fixed oils are conventionally employed as solvents or suspending mediums. For this purpose, any bland fixed oil may be employed including synthetic mono- or di-glycerides. Fatty acids such as oleic acid and its glyceride derivatives, including olive oil and castor oil, especially in their polyoxyethylated versions, are useful in the preparation of injectables. These oil solutions or suspensions may also contain long-chain alcohol diluents or dispersants.

The compounds of this invention may also be administered rectally in the form of suppositories. These compositions can be prepared by mixing the drug with a suitable non-irritating excipient which is solid at room temperature, but liquid at rectal temperature and, therefore, will melt in the rectum to release the drug. Such materials include cocoa butter, beeswax and polyethylene glycols.

The compounds of this invention may also be administered topically, especially when the conditions addressed for treatment involve areas or organs readily accessible by topical application, including neurological disorders of the eye, the skin, or the lower intestinal tract. Suitable topical formulations are readily prepared for each of these areas.

For topical application to the eye, or ophthalmic use, the compounds can be formulated as micronized suspensions in isotonic, pH adjusted sterile saline, or, preferably, as solutions in isotonic, pH adjusted sterile saline, either with or without a preservative such as benzylalkonium chloride. Alternatively for the ophthalmic uses the compounds may be formulated in an ointment such as petrolatum.

For topical application to the skin, the compounds can be formulated in a suitable ointment containing the compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, the compounds can be formulated in a suitable lotion or cream containing the active compound suspended or dissolved in, for example, a mixture of one or more of the following: mineral oil, sorbitan monostearate, polysorbate 60, cetyl esters wax, cetearyl alcohol, 2-octyldodecanol, benzyl alcohol and water.

Topical application for the lower intestinal tract an be effected in a rectal suppository formulation (see above) or in a suitable enema formulation.

Dosage levels on the order of about 0.1 mg to about 10,000 mg of the active ingredient compound are useful in the treatment of the above conditions, with preferred levels of about 0.1 mg to about 1,000 mg. The amount of active ingredient that may be combined with the carrier materials to produce a single dosage form will vary depending upon the host treated and the particular mode of administration. Typically, in vitro dosage-effect results provide useful guidance on the proper doses for patient administration. Studies in animal models are also helpful. The considerations for determining the proper dose levels are well known in the art.

It is understood, however, that a specific dose level for any particular patient will depend upon a variety of factors including the activity of the specific compound employed, the age, body weight, general health, sex, diet, time of administration, rate of excretion, drug combination, and the severity of the particular disease being treated and form of administration.

To effectively treat alopecia or promote hair growth, the compounds used in the inventive methods and pharmaceutical compositions must readily affect the targeted areas. For these purposes, the compounds are preferably administered topically to the skin.

For topical application to the skin, the compounds can be formulated into suitable ointments containing the compounds suspended or dissolved in, for example, mixtures with one or more of the following: mineral oil, liquid petrolatum, white petrolatum, propylene glycol, polyoxyethylene polyoxypropylene compound, emulsifying wax and water. Alternatively, the compounds can be formulated into suitable lotions or creams containing the active compound suspended or dissolved in, for example, a mixture of one or more of the following: mineral oil, sorbitan monostearate, polysorbate 60, cetyl ester wax, cetearyl alcohol, 2-octyldodecanol, benzyl alcohol and water.

The compounds can be administered with other hair revitalizing agents. Specific dose levels for the other hair revitalizing agents will depend upon the factors previously stated and the effectiveness of the drug combination. Other routes of administration known in the pharmaceutical art are also contemplated by this invention.

Specific embodiments of the inventive compounds are presented in Tables I, II, and III. The present invention contemplates employing the compounds of Tables I, II and III, below, for use in compositions and methods to prevent and/or treat a neurological disorder in an animal, and for use in compositions and methods to treat alopecia and promote hair growth in an animal, and all other uses suggested in this specification.

**Table II.**

| | | | | | |
|---|---|---|---|---|---|
| 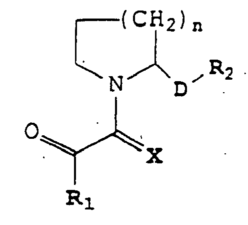 | | | | | |

| No. | X | n | R₁ | D | R₂ |
|---|---|---|---|---|---|
| 26 | O | 1 | 1,1-dimethyl propyl | CH₂ | OH |
| 27 | O | 1 | 1,1-dimethyl propyl | bond | SO₃H |
| 28 | O | 1 | 1,1-dimethyl propyl | CH₂ | CN |
| 29 | O | 1 | 1,1-dimethyl propyl | bond | CN |
| 30 | O | 1 | 1,1-dimethyl propyl | bond | tetrazolyl |
| 34 | O | 1 | 1,1-dimethyl propyl | bond | CONH₂ |
| 35 | O | 2 | 1,1-dimethyl propyl | bond | CONH₂ |
| 43 | O | 2 | 3,4,5- trimethoxyphenyl | CH₂ | tetrazolyl |

**TABLE III**

| | | | | | |
|---|---|---|---|---|---|
| 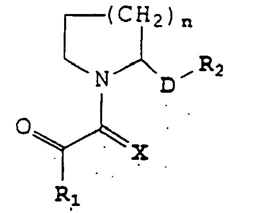 | | | | | |

| **No.** | **n** | **X** | **D** | **R₂** | **R₁** |
|---|---|---|---|---|---|
| 47 | 1 | O | bond | Tetrazole | Benzyl |
| 48 | 1 | O | bond | SO₂H | α-MethylBenzyl |
| 51 | 1 | O | bond | CN | α-MethylBenzyl |
| 101 | 1 | O | (CH₂)₂ | CN | 1,1-dimethylpropyl |
| 102 | 1 | O | (CH₂)₃ | CN | 1,1-dimethylpropyl |
| 118 | 2 | O | bond | 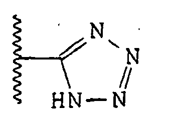 | 1,1-dimethylpropyl |

Additional claimed or comparative carboxylic acids and isosteres of N-heterocyclic compounds which also show the remarkable neurotrophic and hair growth effects of the present invention are shown below in Table IV:

**TABLE IV**

| | | | | | |
|---|---|---|---|---|---|
| 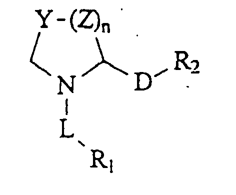 | | | | | |

| Cpd. | n | D | R₂ | L | R₁ |
|---|---|---|---|---|---|
| ltr/number | | | | | |
| A/137 | 1 | bond | COOH | 1,2-dioxoethyl | 1,1-dimethylpropyl |
| B/138 | 2 | bond | COOH | 1,2-dioxoethyl | 1,1-dimethylpropyl |
| C | 1 | bond | COOH | SO₂ | Benzyl |
| D/26 | 1 | CH₂ | OH | 1,2-dioxoethyl | 1,1-dimethylpropyl |
| E/30 | 1 | bond | tetrazole | 1,2-dioxoethyl | 1,1-dimethylpropyl |
| F/29 | 1 | bond | -CN | 1,2-dioxoethyl | 1,1-dimethylpropyl |
| G/35 | 2 | bond | CONH₂ | 1,2-dioxoethyl | 1,1-dimethylpropyl |
| where Y and Z are both carbon for compounds A-G, | | | | | |
| H | 1 | bond | COOH | 1,2-dioxoethyl | 1,1-dimethylpropyl |
| I | 1 | bond | COOH | 1,2-dioxoethyl | 1,1-dimethylpropyl |

where Z is S for compound H and
where Y is S for compound I.

### Pharmaceutical Compositions of the Present Invention

The present invention relates to a pharmaceutical composition comprising:
(i) an effective amount of an N-heterocyclic carboxylic acid or carboxylic acid isostere compound; and
(ii) a pharmaceutically acceptable carrier.

The present invention also relates to a pharmaceutical composition comprising:
(i) an effective amount of an N-heterocyclic carboxylic acid or carboxylic acid isostere compound for treating neurodegenerative diseases, neurological disorders, and nerve damage, or promoting nerve growth in an animal; and
(ii) a pharmaceutically acceptable carrier.

The present invention also relates to a pharmaceutical composition comprising:
(i) an effective amount of an N-heterocyclic carboxylic acid isostere compound for treating alopecia or promoting hair growth in an animal; and
(ii) a pharmaceutically acceptable carrier.

The compounds can be administered with other neurotrophic agents such as neurotrophic growth factor, brain derived growth factor, glial derived growth factor, cilial neurotrophic factor, insulin growth factor and active truncated derivatives thereof, acidic fibroblast growth factor, basic fibroblast growth factor, platelet-derived growth factors, neurotropin-3 and neurotropin 4/5. The dosage level of other neurotrophic drugs will depend upon the factors previously stated and the neurotrophic effectiveness of the drug combination.

### Methods of the Present Invention

The present invention relates to the use of any of the compounds seen in Tables I, II, III, IV, and other compounds embodied herein, in the preparation of a medicament for the treatment of a disease such as peripheral neuropathy caused by physical injury or disease state, physical damage to the brain, physical damage to the spinal cord, stroke associated with brain damage, Alzheimer's Disease, Parkinson's Disease, and amyotrophic lateral sclerosis. The present invention also relates to the use of carboxylic acid and carboxylic acid isostere compounds for treating the above-mentioned neuropathies, neurological disorders, and neurological damage.

The present invention also relates to using the inventive compounds and compositions in the preparation of a medicament for the treatment of alopecia or promoting hair growth in an animal.

The inventive compounds are particularly useful for treating male pattern alopecia, alopecia senilis, alopecia areata, alopecia resulting from skin lesions or tumors, alopecia resulting from cancer therapy such as chemotherapy and radiation, and alopecia resulting from systematic disorders such as nutritional disorders and internal secretion disorders.

It is understood, however, that a specific dose level for any particular patient will depend upon a variety of factors including the activity of the specific compound employed, the age, body weight, general health, sex, diet, time of administration, rate of excretion, drug combination, and the severity of the particular disease or disorder being treated and form of administration.

### MPTP Model of Parkinson's Disease in Mice

MPTP lesioning of dopaminergic neurons in mice was used as an animal model of Parkinson's Disease. Four week old male CDl white mice were dosed i.p. with 30 mg/kg of MPTP for 5 days. The inventive compounds (4 mg/kg), or vehicle, were administered s.c. along with the MPTP for 5 days, as well as for an additional 5 days following cessation of MPTP treatment. At 18 days following MPTP treatment, the animals were sacrificed and the striata were dissected and homogenized. Immunostaining was performed on saggital and coronal brain sections using anti-tyrosine hydoxylase Ig to quantitate survival and recovery of dopaminergic neurons. In animals treated with MPTP and vehicle, a substantial loss of functional dopaminergic terminals was observed as compared to non-lesioned animals. In another protocol, test compounds were administered only subsequent to MPTP-induced lesioning. Thus, after animals were treated with MPTP for 5 days, an additional 3 days passed before beginning oral drug treatment on day 8. Animals were treated with the inventive compounds (0.4 mg/kg), administered orally, once a day for 5 days total. On day 18, the animals were sacrificed and analyzed as described above.

Table V presents the percent recovery of dopaminergic neurons in the first (concurrent dosing) paradigm in animals receiving carboxylic acid or carboxylic acid isostere compounds of the present invention.

Table V, below, shows the remarkable neuroregenerative effects of the inventive carboxylic acid or carboxylic acid isostere related compounds illustrating the neurotrophic capability of carboxylic acid isosteres as a class showing that lesioned animals receiving the carboxylic acid or carboxylic acid isostere compounds provide a remarkable recovery of TH-stained dopaminergic neurons.

**Table V - MPTP Neurodegenerative Model**

| | % Recovery |
|---|---|
| Compound A | 26.7 % |
| Compound B | ND |
| Compound C | 24.4 % |
| Compound D | 23.2 % |
| Compound E | 19.6 % |
| Compound F | 34.1 % |
| Compound G | 46.5 % |
| Compound H | 14.0 % |
| Compound I | ND |

Percent striatal innervation density was quantitated in brain sections with an anti-tyrosine hydroxylase immunoglobulin, which is indicative of functional dopaminergic neurons. The striatal innervation density of 23% for animals pretreated with only a vehicle and administered a vehicle orally during treatment, is indicative of normal non-lesioned striatal tissue. Striatal innervation density is reduced to 5% for animals pretreated with MPTP and administered a vehicle orally during treatment, and is indicative of MPTP-induced lesioning. Surprisingly, striatal innervation density is increased 8-13% for animals pretreated with MPTP and administered 0.4 mg/kg of an inventive compound orally during treatment, indicating substantial neuronal regeneration after induction of MPTP-derived lesions.

The following examples are illustrative of preferred embodiments of the invention and are not to be construed as limiting the invention thereto. All polymer molecular weights are mean average molecular weights. All percentages are based on the percent by weight of the final delivery system or formulation prepared unless otherwise indicated and all totals equal 100% by weight.

### EXAMPLES

The inventive compounds may be prepared by a variety of synthetic sequences that utilize established chemical transformations. A general pathway to the present compounds is described in Scheme I. N-glyoxylproline derivatives may be prepared by reacting L-proline methyl ester with methyl oxalyl chloride as shown in Scheme I. The resulting oxamates may be reacted with a variety of carbon nucleophiles to obtain compounds used in the present invention.

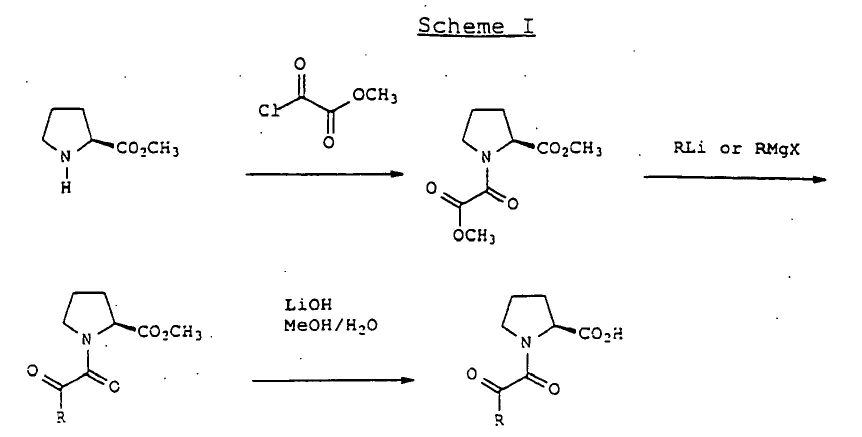

### EXAMPLE 1 (compound 137) (not a compound according to the present invention)

### Synthesis of (2S)-1-(3,3-dimezhyl-1,-dioxopentyl)-2-pyrrolidinecarboxylate.

### a. Synthesis of (2S)-1-(1,2-dioxo-2-methoxyethyl)-2-pyrrolidinecarboxylate.

A solution of L-proline methyl ester hydrochloride (3.08 g; 18.60 mmol) in dry methylene chloride was cooled to 0°C and treated with triethylamine (3.92 g; 38.74 mmol; 2.1 eq). After stirring the formed slurry under a nitrogen atmosphere for 15 min, a solution of methyl oxalyl chloride (3.20 g; 26.12 mmol) in methylene chloride (45 mL) was added dropwise. The resulting mixture was stirred at 0°C for 1.5 hr. After filtering to remove solids, the organic phase was washed with water, dried over MgSO₄ and concentrated. The crude residue was purified on a silica gel column, eluting with 50% ethyl acetate in hexane, to obtain 3.52 g (88%) of the product as a reddish oil. Mixture of cis-trans amide rotamers; data for trans rotamer given. ¹H NMR (CDCl₃): δ 1.93 (dm, 2H); 2.17 (m, 2H); 3.62 (m, 2H); 3.71 (s, 3H); 3.79, 3.84 (s, 3H total); 4.86 (dd, 1H, J = 8.4, 3.3).

### b. Synthesis of methyl (2S)-1-(1,2-dioxo-3,3-dimethylpentyl)-2-pyrrolidinecarboxylate.

A solution of methyl (2S)-1-(1,2-dioxo-2-methoxyethyl)-2-pyrrolidinecarboxylate (2.35 g; 10.90 mmol) in 30 mL of tetrahydrofuran (THF) was cooled to - 78°C and treated with 14.2 mL of a 1.0 M solution of 1,1-dimethylpropylmagnesium chloride in THF. After stirring the resulting homogeneous mixture at -78°C for three hours, the mixture was poured into saturated ammonium chloride (100 mL) and extracted into ethyl acetate. The organic phase was washed with water, dried, and concentrated, and the crude material obtained upon removal of the solvent was purified on a silica gel column, eluting with 25% ethyl acetate in hexane, to obtain 2.10 g (75%) of the oxamate as a colorless oil. ¹H NMR (CDCl₃): δ 0.88 (t, 3H); 1.22, 1.26 (s, 3H each); 1.75 (dm, 2H); 1.87-2.10 (m, 3H); 2.23 (m, 1H); 3.54 (m, 2H); 3.76 (s, 3H); 4.52 (dm, 1H, J = 8.4, 3.4).

### c. Synthesis of (2S)-1-(1,2-dioxo-3,3-dimethylpentyl)-2-pyrrolidinecarboxylic acid (Compound 137).

A mixture of methyl (2S)-1-(1,2-dioxo-3,3-dimethylpentyl)-2-pyrrolidinecarboxylate (2.10 g; 8.23 mmol), 1 N LiOH (15 mL), and methanol (50 mL) was stirred at 0°C for 30 min and at room temperature overnight. The mixture was acidified to pH 1 with 1 N HCl, diluted with water, and extracted into 100 mL of methylene chloride. The organic extract was washed with brine and concentrated to deliver 1.73 g (87%) of snow-white solid which did not require further purification. ¹H NMR (CDCl₃): δ 0.87 (t, 3H); 1.22, 1.25 (s, 3H each); 1.77 (dm, 2H); 2.02 (m, 2H); 2.17 (m, 1H); 2.25 (m, 1H); 3.53 (dd, 2H, J = 10.4, 7.3); 4.55 (dd, 1H, J = 8.6, 4.1). 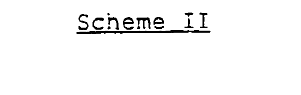 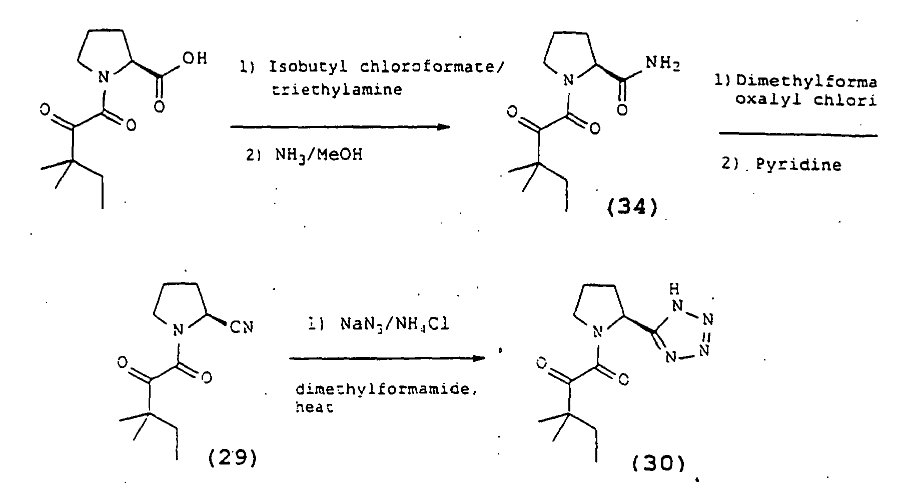 Inventive compounds containing bridged rings may be synthesized using the above synthetic schemes by substituting the substrates containing the N-heterocyclic ring structures with comparable substrates containing bridged ring structures.

### EXAMPLE 2

### Synthesis of (2S)-1-(1,2-dioxo-3,3-dimethylpentyl)-2-pyrrolidinecarboxamide. (Compound 34)

Isobutyl chloroformate (20 mmol, 2.7 mL) was added to a solution containing (2S)-1-(1,2-dioxo-3,3-cimethylpentyl)-2-pyrrolidinecarboxylic acid (4.89 g, 20 mmol)(from Example 1) in 50 mL methylene chloride at -10°C with stirring. After 5 minutes, ammonia was added dropwise (20 mmol, 10 mL of 2 M ethyl alcohol solution). The reaction was warmed up to room temperature after stirring at -10°C for 30 minutes. The mixture was diluted with water, and extracted into 200 mL methylene chloride. The organic extract was concentrated and further purified by silica gel to give 4.0 g of product as a white solid (81.8% yield). ¹H NMR (CDCl₃): δ 0.91 (t, 3H, J= 7.5); 1.28 (s, 6H, each); 1.63-1.84 (m, 2H); 1.95-2.22 (m, 3H); 2.46 (m, 1H) ; 3.55-3.67 (m, 2H); 4.67 (t, 1H, J= 7.8); 5.51-5.53 (br, 1H, NH); 6.80 (br, 1H, NH).

### EXAMPLE 3

### Synthesis of (2S)-1-(1,2-dioxo-3,3-dimethylpentyl)-2-pyrrolidinecarbonitrile. (Compound 29)

To a solution of 0.465 mL DMF (6 mmol) in 10 mL acetonitrile at 0°C was added 0.48 mL (5.5 mmol) of oxalyl chloride. A white precipitate formed immediately and was accompanied by gas evolution. When complete, a solution of 1.2 g (5 mmol) of (2S)-1-(1,2-dioxo-3,3-dimethylpentyl)-2-pyrrolidinecarboxamide (from Example 2) in 2.5 mL acetonitrile was added. When the mixture became homogeneous, 0.9 mL (11 mmol) pyridine was added. After 5 min., the mixture was diluted into water and extracted by 200 mL ethyl acetate. The organic layer was concentrated and further purified by silica gel to give 0.8 g product as a white solid (72% yield). ¹H NMR (CDCl₃): δ 0.87 (t, 3H, J= 7.5); 1.22 (s, 3H); 1.24 (s, 3H); 1.80 (m, 2H); 2.03-2,.23 (m, 4H); 3.55 (m, 2H); 4.73 (m, 1H).

### EXAMPLE 4

### Synthesis of (2S)-1-(1,2-dioxo-3,3-dimethylpentyl)-2-pyrrolidinetetrazole. (Compound 30)

A mixture of (2S)-1-(1,2-dioxo-3,3-dimethylpentyl)-2-pyrrolidinecarbonitrile (222 mg, 1 mmol) (from Example 3), NaN₃ (81 mg, 1.3 mmol) and NH₄Cl (70 mg, 1.3 mmol) in 3 mL DMF was stirred at 130°C for 16 hours. The mixture was concentrated and purified by silica gel to afford 200 mg product as white solid (75.5% yield). ¹H NMR (CDCl₃): δ 0.88 (t, 3H, J= 7.5); 1.22 (s, 6H); 1.68 (m, 2H); 2.05-2.36 (m, 3H); 2.85 (m, 1H); 3.54 (m, 1H); 3.75 (m, 1H); 5.40 (m, 1H).

### Example 5

### In Vivo Hair Generation Test With C57 Black 6 Mice

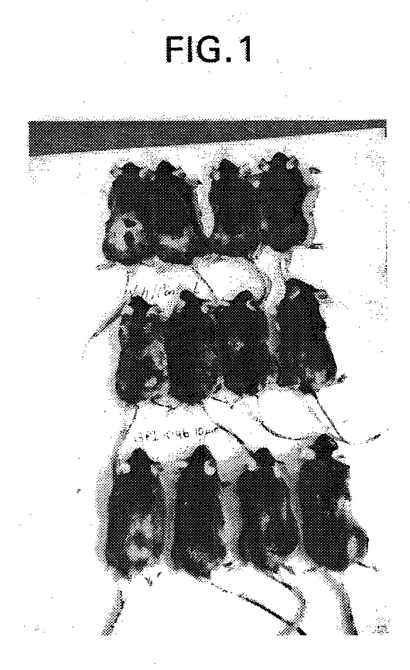

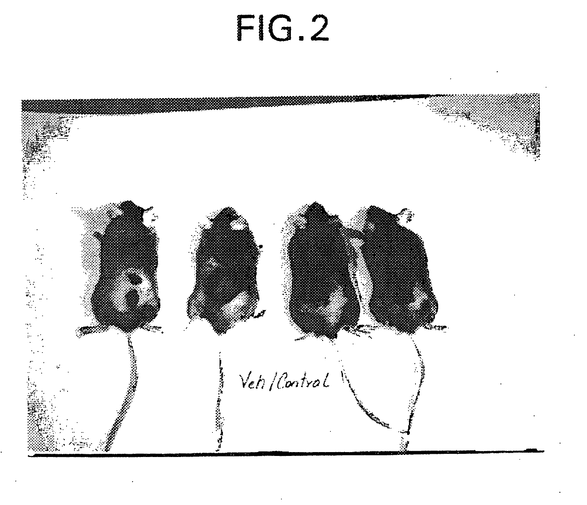

C57 black 6 mice were used to demonstrate the hair revitalizing properties of the N-heterocyclic carboxylic acids or carboxylic acid isosteres. Referring now to FIGS. 1 and 2 of the drawings, C57 black 6 mice, approximately 7 weeks old, had an area of about 2 inches by 2 inches on their hindquarters shaved to remove all existing hair. Care was taken not to nick or cause abrasion to the underlaying dermal layers. The animals were in anagen growth phase, as indicated by the pinkish color of the skin. Referring now to FIG. 2, four animals per group were treated by topical administration with 20% propylene glycol vehicle (FIG. 2), or neuroimmunophilin FKBP ligands dissolved in the vehicle. The animals were treated with vehicle or neuroimmunophilin ligands every 48 hours (3 applications total over the course of 5 days) and the hair growth was allowed to proceed for 6 weeks. Hair growth was quantitated by the percent of shaved area covered by new hair growth during this time period.

FIG. 2 shows that animals treated with vehicle exhibited only a small amount of hair growth in patches or tufts, with less than 3% of the shaved area covered with new growth.

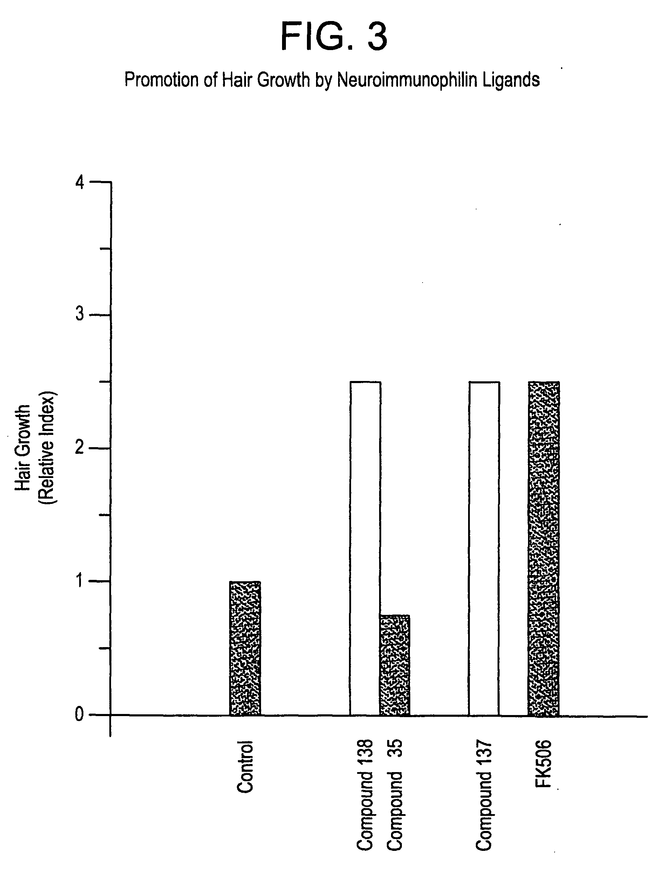

In contrast, FIG. 3 shows that animals treated for 2 weeks with the N-heterocyclic carboxylic acid compounds i.e. compound A (137), compound B (138), and compound G (35) exhibited dramatic hair growth, covering greater than 25% of the shaved area in all animals for two of the compounds.

FIG. 3 shows the relative hair growth on shaven C57 black 6 mice 14 days after being treated with one of three N-heterocyclic carboxylic acids or carboxylic acid isosteres. The mice had a 2 x 2 inch region on their backside shaved to remove all hair. Care was taken not to nick or cause abrasion to the underlying dermal layers. Compounds at a concentration of 1 µmole per milliliter were carefully applied to the shaved area of the mice (5 mice per group) three times per week. Hair growth was evaluated 14 days after initiation of drug treatment. The relative scale for assessing hair growth is as follows:
0 = no growth;
1 = beginning of growth in small tufts;
2 = hair growth covering over <25% of shaved area;
3 = hair growth covering over >25% of shaved area, but less than 50% of shaved area;
4 = hair growth covering over >50% of shaved area, but less than 75% of shaved area;
5 = complete hair growth of shaved area.

### Example 6

A lotion comprising the following composition may be prepared.

| | (%) |
|---|---|
| 95% Ethanol | 80.0 |
| an N-heterocyclic carboxylic acid isostere | 10.0 |
| α-Tocopherol acetate | 0.01 |
| Ethylene oxide (40 mole) adducts of hardened castor oil | 0.5 |
| purified water | 9.0 |
| perfume and dye | q.s. |

Into 95% ethanol are added an N-heterocyclic carboxylic acid isostere, α-tocopherol acetate, ethylene oxide (40 mole) adducts of hardened castor oil, perfume and a dye. The resulting mixture is stirred and dissolved, and purified water is added to the mixture to obtain a transparent liquid lotion.

5 mL of the lotion may be applied once or twice per day to a site having marked baldness or alopecia.

### Example 7

A lotion comprising the following composition shown may be prepared.

| | (%) |
|---|---|
| 95% Ethanol | 80.0 |
| an N-heterocyclic carboxylic acid isostere | 0.005 |
| Hinokitol | 0.01 |
| Ethylene oxide (40 mole) adducts of hardened castor oil | 0.5 |
| Purified water | 19.0 |
| Perfume and dye | q.s. |

Into 95% ethanol are added an N-heterocyclic carboxylic acid isostere, hinokitol, ethylene oxide (40 mole) adducts of hardened castor oil, perfume, and a dye. The resulting mixture is stirred, and purified water is added to the mixture to obtain a transparent liquid lotion.

The lotion may be applied by spraying once to 4 times per day to a site having marked baldness or alopecia.

### Example 8

An emulsion may be prepared from A phase and B phase having the following compositions.

| **(A phase)** | (%) |
|---|---|
| Whale wax | 0.5 |
| Cetanol | 2.0 |
| Petrolatum | 5.0 |
| Squalane | 10.0 |
| Polyoxyethylene (10 mole) monostearate | 2.0 |
| Sorbitan monooleate | 1.0 |
| an N-heterocyclic carboxylic acid isostere | 0.01 |

| **(B phase)** | (%) |
|---|---|
| Glycerine | 10.0 |
| Purified water | 69.0 |
| Perfume, dye, and preservative | q.s. |

The A phase and the B phase are respectively heated and melted and maintained at 80°C. Both phases are then mixed and cooled under stirring to normal temperature to obtain an emulsion.

The emulsion may be applied by spraying once to four times per day to a site having marked baldness or alopecia.

### Example 9

A cream may be prepared from A phase and B phase having the following compositions.

| **(A Phase)** | (%) |
|---|---|
| Fluid paraffin | 5.0 |
| Cetostearyl alcohol | 5.5 |
| Petrolatum | 5.5 |
| Glycerine monostearate | 33.0 |
| Polyoxyethylene (20 mole) 2-octyldodecyl ether | 3.0 |
| Propylparaben | 0.3 |

| **(B Phase)** | (%) |
|---|---|
| an N-heterocyclic carboxylic acid isostere | 0.8 |
| Glycerine | 7.0 |
| Dipropylene glycol | 20.0 |
| Polyethylene glycol 4000 | 5.0 |
| Sodium Hexametaphosphate | 0.005 |
| Purified water | 44.895 |

The A phase is heated and melted, and maintained at 70°C. The B phase is added into the A phase and the mixture is stirred to obtain an emulsion. The emulsion is then cooled to obtain a cream.

The cream may be applied once to 4 times per day to a site having marked baldness or alopecia.

### Example 10

A liquid comprising the following composition may be prepared.

| | (%) |
|---|---|
| Polyoxyethylene butyl ether | 20.0 |
| Ethanol | 50.0 |
| an N-heterocyclic carboxylic acid isostere | 0.001 |
| Propylene glycol | 5.0 |
| Polyoxyethylene hardened castor oil derivative (ethylene oxide 80 mole adducts) | 0.4 |
| Perfume | q.s. |
| Purified water | q.s. |

Into ethanol are added polyoxypropylene butyl ether, propylene glycol, polyoxyethylene hardened castor oil, an N-heterocyclic carboxylic acid isostere, and perfume. The resulting mixture is stirred, and purified water is added to the mixture to obtain a liquid.

The liquid may be applied once to 4 times per day to a site having marked baldness or alopecia.

### Example 11

A shampoo comprising the following composition may be prepared.

| | (%) |
|---|---|
| Sodium laurylsulfate | 5.0 |
| Triethanolamine laurylsulfate | 5.0 |
| Betaine lauryldimethylaminoacetate | 6.0 |
| Ethylene glycol distearate | 2.0 |
| Polyethylene glycol | 5.0 |
| an N-heterocyclic carboxylic acid isostere | 5.0 |
| Ethanol | 2.0 |
| Perfume | 0.3 |
| Purified water | 69.7 |

Into 69.7 of purified water are added 5.0 g of sodium laurylsulfate, 5.0 g of triethanolamine laurylsulfate, 6.0 g of betaine lauryldimethyl-aminoacetate. Then a mixture obtained by adding 5.0 g of an N-heterocyclic carboxylic acid isostere, 5.0 g of polyethylene glycol, and 2.0 g of ethylene glycol distearate to 2.0 g of ethanol, followed by stirring, and 0.3 g of perfume are successively added. The resulting mixture is heated and subsequently cooled to obtain a shampoo.

The shampoo may be used on the scalp once or twice per day.

### Example 12

A patient is suffering from alopecia senilis. An N-heterocyclic carboxylic acid isostere, or a pharmaceutical composition comprising the same, may be administered to the patient. Increased hair growth is expected to occur following treatment.

### Example 13

A patient is suffering from male pattern alopecia. An N-heterocyclic carboxylic acid isostere, or a pharmaceutical composition comprising the same may be administered to the patient. Increased hair growth is expected to occur following treatment.

### Example 14

A patient is suffering from alopecia areata. An N-heterocyclic carboxylic acid isostere, or a pharmaceutical composition comprising the same, may be administered to the patient. Increased hair growth is expected to occur following treatment.

### Example 15

A patient is suffering from hair loss caused by skin lesions. An N-heterocyclic carboxylic acid isostere, or a pharmaceutical composition comprising the same, may be administered to the patient. Increased hair growth is expected to occur following treatment.

### Example 16

A patient is suffering from hair loss caused by tumors. An N-heterocyclic carboxylic acid isostere, or a pharmaceutical composition comprising the same, may be administered to the patient. Increased hair growth is expected to occur following treatment.

### Example 17

A patient is suffering from hair loss caused by a systematic disorder, such as a nutritional disorder or an internal secretion disorder. An N-heterocyclic carboxylic acid isostere, or a pharmaceutical composition comprising the same, may be administered to the patient. Increased hair growth is expected to occur following treatment.

### Example 18

A patient is suffering from hair loss caused by chemotherapy. An N-heterocyclic carboxylic acid isostere, or a pharmaceutical composition comprising the same, may be administered to the patient. Increased hair growth is expected to occur following treatment.

### Example 19

A patient is suffering from hair loss caused by radiation. An N-heterocyclic carboxylic acid isostere, or a pharmaceutical composition comprising the same may, be administered to the patient. Increased hair growth is expected to occur following treatment.

### Example 20

A patient is suffering from a neurodegenerative disease. A carboxylic acid isostere of an N-heterocyclic ring or a pharmaceutical composition comprising the same is administered. It would be expected that the patient would improve their condition or recover.

### Example 21

A patient is suffering from a neurological disorder. A carboxylic acid isostere of an N-heterocyclic ring or pharmaceutical compositions comprising same is administered. It would be expected that the patient would improve their condition or recover.

### Example 22

A patient is suffering from stroke. A carboxylic acid isostere of an N-heterocyclic ring or pharmaceutical compositions comprising same is administered. It would be expected that the patient would improve their condition or recover.

### Example 23

A patient is suffering from Parkinson's Disease. A carboxylic acid isostere of an N-heterocyclic ring or pharmaceutical compositions comprising same is administered. It would be expected that the patient would improve their condition or recover.

### Example 24

A patient is suffering from Alzheimer's Disease. A carboxylic acid isostere of an N-heterocyclic ring or pharmaceutical compositions comprising same is administered. It would be expected that the patient would improve their condition or recover.

### Example 25

A patient is suffering from a peripheral neuropathy. A carboxylic acid isostere of an N-heterocyclic ring or pharmaceutical compositions comprising same is administered. It would be expected that the patient would improve their condition or recover.

### Example 26

A patient is suffering from amyotrophic lateral sclerosis. A carboxylic acid isostere of an N-heterocyclic ring or pharmaceutical compositions comprising same is administered. It would be expected that the patient would improve their condition or recover.

### Example 27

A patient is suffering from a spinal injury. A carboxylic acid isostere of an N-heterocyclic ring or pharmaceutical compositions comprising same is administered. It would be expected that the patient would improve their condition or recover.

### Example 28

A patient is at risk of suffering from a neurodegenerative disease or neurological disorder. A carboxylic acid isostere of an N-heterocyclic ring or a pharmaceutical composition comprising the same is prophelactically administered. It would be expected that the patient would be prevented from some or all of the effects of the disease or disorder, or would significally improve their condition or recover over patients who were not pre-treated.

The invention being thus described, it will be obvious that the same may be varied in many ways. Such variations are not to be regarded as a departure from the spirit and scope of the invention and all such modification are intended to be included within the scope of the following claims.

## Claims

1. A compound having the formula (I): 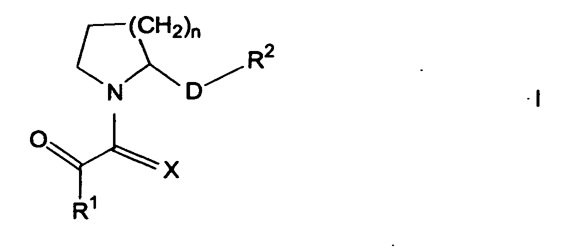 or a pharmaceutically acceptable salt, ester, or solvate of the compound, wherein:
n is 1-3;
X is either 0 or S;
R¹ is C₁-C₉ straight or branched chain alkyl, or C₂-C₉ straight or branched chain alkenyl,
D is a bond, C₁-C₁₀ straight or branched chain alkylene, C₂-C₁₀ alkenylene, or C₂-C₁₀ alkynylene; and
R² is tetrazolyl, -CONH₂, -CN, -SO₃H or -OH;
wherein said alkyl, alkenyl, alkenylene and alkynylene are optionally substituted with one or more substituents independently selected from hydroxy, halo, C₁-C₆ haloalkyl, thiocarbonyl, C₁-C₆ alkoxy, C₂-C₆ alkenoxy, cyano, nitro, imino, C₁-C₆ alkylamino, C₁-C₆ aminoalkyl, sulfhydryl, C₁-C₆ thioalkyl, C₁-C₆ alkylthio, sulfonyl, C₁-C₆ straight or branched chain alkyl, C₂-C₆ straight or branched chain alkenyl or alkynyl, or CO₂R⁷ where R⁷ is hydrogen, C₁-C₉ straight or branched chain alkyl or C₂-C₉ straight or branched chain alkenyl;
**provided that:**
**when** n=1, X is O, D is a bond, and R² is -CONH₂,
**then** R¹ is not methyl, ethyl, iso-propyl, iso-butyl, iso-pentyl, or 4-methylpentyl; and
**further provided that:**
**when** n=1, X is O, D is a bond, and R² is cyano,
**then** R¹ is not methyl.

2. A compound according to claim 1, wherein the values of n, X, R¹, D and R² in formula I fulfill one of the following combinations:
| n | X | R¹ | D | R² |
|---|---|---|---|---|
| 1 | O | 1,1-dimethyl propyl | CH₂ | OH |
| 1 | O | 1,1-dimethyl propyl | bond | SO₃H |
| 1 | O | 1,1-dimethyl propyl | CH₂ | CN |
| 1 | O | 1,1-dimethyl propyl | bond | CN |
| 1 | O | 1,1-dimethyl propyl | bond | tetrazolyl |
| 1 | O | 1,1-dimethyl propyl | bond | CONH₂ |
| 2 | O | 1,1-dimethyl propyl | bond | CONH₂ |
| 1 | O | 1,1-dimethyl propyl | (CH₂)₂ | CN |
| 1 | O | 1,1-dimethyl propyl | (CH₂)₃ | CN |
| 2 | O | 1,1-dimethyl propyl | bond | tetrazolyl |

3. A pharmaceutical composition comprising:
a) an effective amount of a compound as defined in claim 1 or claim 2; and
b) a pharmaceutically acceptable carrier.

4. A pharmaceutical composition according to claim 3, wherein n is 1 in formula I of the compound defined in claim 1.

5. A pharmaceutical composition according to claim 3 or claim 4, further comprising an additional neurotrophic factor.

6. A pharmaceutical composition according to claim 5, wherein said additional neurotrophic factor is selected from neurotrophic growth factor, brain derived growth factor, glial derived growth factor, cilial neurotrophic factor, insulin growth factor and active truncated derivatives thereof, acidic fibroblast growth factor, basic fibroblast growth factor, platelet-derived growth factors, neurotropin-3, and neurotropin 4/5.

7. A pharmaceutical composition according to any of claims 3-6, wherein the compound defined in claim 1 or claim 2 is non-immunosuppressive.

8. Use of a compound as defined in claim 1 or claim 2 for the preparation of a medicament for treating a neurological disorder in an animal, for stimulating growth of damaged peripheral nerves, for promoting neuronal regeneration, and/or for preventing neurodegeneration in the animal.

9. The use of claim 8, wherein the neurological disorder is selected from peripheral neuropathies caused by physical injury or disease state, physical damage to the brain, physical damage to the spinal cord, stroke associated with brain damage, and neurological disorders relating to neurodegeneration.

10. The use of claim 8, wherein the neurological disorder is selected from Alzheimer's Disease, Parkinson's Disease, amyotrophic lateral sclerosis, trigeminal neuralgia, glossopharyngeal, neuralgia, Bell's Palsy, myasthenia gravis, muscular dystrophy, progressive muscular atrophy, progressive bulbar inherited muscular atrophy, herniated, ruptured, or prolapsed invertebrate disk syndromes, cervical spondylosis, plexus disorders, thoracic outlet destruction syndromes, Gullain-Barré syndrome, and sciatic nerve lesions.

11. The use of claim 10, wherein the neurological disorder is Alzheimer's disease.

12. The use of claim 10, wherein the neurological disorder is Parkinson's disease.

13. The use of claim 10, wherein the neurological disorder is amyotrophic lateral sclerosis.

14. Use of a compound as defined in claim 1 or claim 2 for the preparation of a medicament for treating alopecia and/or for promoting hair growth in an animal.

## Patentansprüche

1. Verbindung mit der Formel (I): 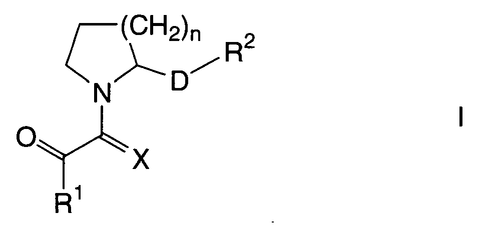 oder ein pharmazeutisch akzeptables/akzeptabler Salz, Ester oder Solvat dieser Verbindung, wobei:
n 1-3 ist;
X entweder O oder S ist;
R¹ ein C₁-C₉ gerad- oder verzweigtkettiges Alkyl oder ein C₂-C₉ gerad- oder verzweigtkettiges Alkenyl ist;
D eine Bindung, ein C₁-C₁₀ gerad- oder verzweigtkettiges Alkylen, C₂-C₁₀-Alkenylen oder C₂-C₁₀-Alkinylen ist; und
R² Tetrazolyl, -CONH₂, -CN, -SO₃H oder -OH ist;
wobei das genannte Alkyl, Alkenyl, Alkenylen und Alkinylen optional mit einem oder mehreren Substituenten substituiert sind, unabhängig voneinander ausgewählt aus Hydroxy, Halo, C₁-C₆-Haloalkyl, Thiocarbonyl, C₁-C₆-Alkoxy, C₂-C₆-Alkenoxy, Cyano, Nitro, Imino, C₁-C₆-Alkylamino, C₁-C₆-Aminoalkyl, Sulfhydryl, C₁-C₆-Thioalkyl, C₁-C₆-Alkylthio, Sulfonyl, C₁-C₆ gerad- oder verzweigtkettiges Alkyl, C₂-C₆ gerad- oder verzweigtkettiges Alkenyl oder Alkinyl, oder CO₂R⁷, wobei R⁷ Wasserstoff, C₁-C₉ gerad- oder verzweigtkettiges Alkyl oder C₂-C₉ gerad- oder verzweigtklettiges Alkenyl ist;
**vorausgesetzt, dass:**
**wenn** n=1, X O ist, D eine Bindung ist und R² -CONH₂ ist,
R¹ nicht Methyl, Ethyl, Isopropyl, Isobutyl, Isonpentyl oder 4-Methylpentyl ist; und
**weiter vorausgesetzt, dass:**
**wenn** n=1, X O ist, D eine Bindung ist und R² Cyano ist,
R¹ nicht Methyl ist.

2. Verbindung gemäß Anspruch 1, wobei die Werte von n, X, R¹, D und R² in Formel I eine der folgenden Kombinationen erfüllen:
| n | X | R¹ | D | R² |
|---|---|---|---|---|
| 1 | O | 1,1-Dimethylpropyl | CH₂ | OH |
| 1 | O | 1,1-Dimethylpropyl | Bindung | SO₃H |
| 1 | O | 1,1-Dimethylpropyl | CH₂ | CN |
| 1 | O | 1,1-Dimethylpropyl | Bindung | CN |
| 1 | O | 1,1-Dimethylpropyl | Bindung | Tetrazolyl |
| 1 | O | 1,1-Dimethylpropyl | Bindung | CONH₂ |
| 2 | O | 1,1-Dimethylpropyl | Bindung | CONH₂ |
| 1 | O | 1,1-Dimethylpropyl | (CH₂)₂ | CN |
| 1 | O | 1,1-Dimethylpropyl | (CH₂)₃ | CN |
| 2 | O | 1,1-Dimethylpropyl | Bindung | Tetrazolyl |

3. Pharmazeutische Zusammensetzung umfassend:
a) eine wirksame Menge einer Verbindung gemäß Anspruch 1 oder 2; und
b) einen pharmazeutisch akzeptablen Träger.

4. Pharmazeutische Zusammensetzung gemäß Anspruch 3, wobei n in der Formel I der Verbindung gemäß Anspruch 1 1 ist.

5. Pharmazeutische Zusammensetzung gemäß Anspruch 3 oder 4, weiterhin umfassend einen zusätzlichen neurotrophen Faktor.

6. Pharmazeutische Zusammensetzung gemäß Anspruch 5, wobei der zusätzliche neurotrophe Faktor ausgewählt ist aus dem neurotrophen Wachstumsfaktor, aus Gehirn stammenden Wachstumsfaktor, aus Glia stammenden Wachstumsfaktor, ciliären neurotrophen Faktor, Insulinwachstumsfaktor und aktiven gekürzten Derivaten davon, sauren Fibroplasten-Wachstumsfaktor, basischen Fibroplasten-Wachstumsfaktor, plättchenabgeleiteten Wachstumsfaktoren, Neurotropin-3 und Neurotropin 4/5.

7. Pharmazeutische Zusammensetzung gemäß einem der Ansprüche 3 bis 6, wobei die Verbindung gemäß Anspruch 1 oder 2 nicht immunosuppressiv ist.

8. Verwendung einer Verbindung gemäß Anspruch 1 oder 2 zur Herstellung eines Medikaments zur Behandlung einer neurologischen Störung bei einem Tier, zur Stimulierung des Wachstums von geschädigten peripheren Nerven, zur Förderung neuronaler Regeneration und/oder zur Prävention von Neurodegeneration bei einem Tier.

9. Verwendung gemäß Anspruch 8, wobei die neurologische Störung ausgewählt ist aus peripheren Neuropathien verursacht durch physische Verletzung oder Krankheitszustand, physischer Schädigung des Gehirns, physischer Schädigung des Rückenmarks, Schlaganfall assoziiert mit Hirnschäden und neurologischen Störungen im Zusammenhang mit Neurodegeneration.

10. Verwendung gemäß Anspruch 8, wobei die neurologische Störung ausgewählt ist aus Alzheimer Krankheit, Parkinson Krankheit, amyotropher lateraler Sklerose, Trigeminus Neuralgie, glossoparyngealer Neuralgie, einseitiger facialer Lähmung (Bell-Lähmung), Myasthenia gravis, muskulärer Dystrophy, progressiver muskulärer Atropie, progressiver vererbter muskulärer Bulberatropie, Bandscheibenhernie, Bandscheibenruptur, Bandscheibenprolaps, cervicale Spondolyse, Plexuserkrankungen, Thoraxauslass-Engpasssyndrom, Gullain-Barre-Syndrom und Ischiasschädigungen.

11. Verwendung gemäß Anspruch 10, wobei die neurologische Störung Alzheimer Krankheit ist.

12. Verwendung gemäß Anspruch 10, wobei die neurologische Störung Parkinson Krankheit ist.

13. Verwendung gemäß Anspruch 10, wobei die neurologische Störung amyotrophe laterale Sklerose ist.

14. Verwendung einer Verbindung gemäß Anspruch 1 oder 2 zur Herstellung eines Medikaments zur Behandlung von Alopezia und/oder zur Förderung des Haarwuchses bei einem Tier.

## Revendications

1. Composé ayant la formule (I): 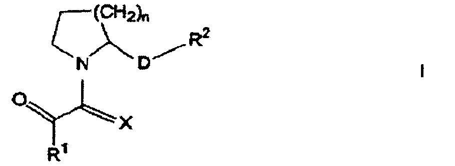 ou sel, ester, ou solvate du composé pharmaceutiquement acceptable, dans lequel :
n prend la valeur de 1 à 3 ;
X représente soit O soit S ;
R¹ représente un groupe alkyle en C₁-C₉ à chaîne linéaire ou ramifiée, ou un groupe alcényle en C₂-C₉ à chaîne linéaire ou ramifiée,
D représente une liaison, un groupe alkylène en C₁-C₁₀ à chaîne linéaire ou ramifiée, un groupe alcénylène en C₂-C₁₀, ou un groupe alcynylène en C₂-C₁₀ ; et
R² représente le groupe tétrazolyle, -CONH₂, -CN, -SO₃H ou -OH ;
dans lequel lesdits groupes alkyle, alcényle, alcénylène et alcynylène sont éventuellement substitués par un ou plusieurs substituant(s) indépendamment choisi(s) parmi un groupe hydroxy, halogéno, halogénoalkyle en C₁-C₆, thiocarbonyle, alcoxy en C₁-C₆, alcénoxy en C₂-C₆, cyano, nitro, imino, alkylamino en C₁-C₆, aminoalkyle en C₁-C₆, sulfhydryle, thioalkyle en C₁-C₆, alkylthio en C₁-C₆, sulfonyle, alkyle en C₁-C₆ à chaîne linéaire ou ramifiée, alcényle ou alcynyle en C₂-C₆ à chaîne linéaire ou ramifiée, ou CO₂R⁷ dans laquelle R⁷ représente un atome d'hydrogène, un groupe alkyle en C₁-C₉ à chaîne linéaire ou ramifiée ou un groupe alcényle en C₂-C₉ à chaîne linéaire ou ramifiée;
**à condition que :**
**lorsque** n = 1, X représente O, D représente une liaison, et R² représente -CONH₂,
**alors** R¹ ne soit pas un groupe méthyle, éthyle, iso-propyle, iso-butyle, iso-pentyle, ou 4-méthylpentyle ; et
**à condition en outre que :**
**lorsque** n = 1, X représente O, D représente une liaison, et R² représente un groupe cyano,
**alors** R¹ ne soit pas un groupe méthyle.

2. Composé selon la revendication 1, dans lequel les valeurs de n, X, R¹, D et R² dans la formule I satisfont l'une des combinaisons suivantes :
| n | X | R¹ | D | R² |
|---|---|---|---|---|
| 1 | O | 1,1-diméthyl propyle | CH₂ | OH |
| 1 | O | 1,1-diméthyl propyle | liaison | SO₃H |
| 1 | O | 1,1-diméthyl propyle | CH₂ | CN |
| 1 | O | 1,1-diméthyl propyle | liaison | CN |
| 1 | O | 1,1-diméthyl propyle | liaison | tétrazolyle |
| 1 | O | 1,1-diméthyl propyle | liaison | CONH₂ |
| 2 | O | 1,1-diméthyl propyle | liaison | CONH₂ |
| 1 | O | 1,1-diméthyl propyle | (CH₂)₂ | CN |
| 1 | O | 1,1-diméthyl propyle | (CH₂)₃ | CN |
| 2 | O | 1,1-diméthyl propyle | liaison | tétrazolyle |

3. Composition pharmaceutique comprenant:
a) une quantité efficace d'un composé tel que défini selon la revendication 1 ou la revendication 2 ; et
b) un support pharmaceutiquement acceptable.

4. Composition pharmaceutique selon la revendication 3, dans laquelle n prend la valeur de 1 dans la formule I du composé défini dans la revendication 1.

5. Composition pharmaceutique selon la revendication 3 ou la revendication 4, comprenant en outre un facteur neurotrophique supplémentaire.

6. Composition pharmaceutique selon la revendication 5, dans laquelle ledit facteur neurotrophique supplémentaire est choisi parmi un facteur de croissance neurotrophique, un facteur de croissance issu du cerveau, un facteur de croissance issu des cellules gliales, un facteur neurotrophique ciliaire, un facteur de croissance analogue à l'insuline, et des dérivés actifs tronqués de ceux-ci, un facteur de croissance des fibroblastes-acide, un facteur de croissance des fibroblastes-basique, des facteurs de croissance dérivés des plaquettes, la neurotropine-3, et la neurotropine 4/5.

7. Composition pharmaceutique selon l'une quelconque des revendications 3 à 6, dans laquelle le composé défini dans la revendication 1 ou la revendication 2 est non-immunosuppresseur.

8. Utilisation d'un composé tel que défini dans la revendication 1 ou la revendication 2, pour la préparation d'un médicament pour un traitement d'un trouble neurologique chez un animal, pour stimuler une croissance de nerfs périphériques endommagés, pour favoriser une régénérescence neuronale, et/ou pour prévenir une neurodégénérescence chez l'animal.

9. Utilisation selon la revendication 8, dans laquelle le trouble neurologique est choisi parmi des neuropathies périphériques provoquées par une blessure physique ou un état pathologique, une lésion physique au niveau du cerveau, une lésion physique au niveau de la moelle épinière, un accident vasculaire cérébral associé à une lésion au niveau du cerveau, et des troubles neurologiques liés à la neurodégénérescence.

10. Utilisation selon la revendication 8, dans laquelle le trouble neurologique est choisi parmi la maladie d'Alzheimer, la maladie de Parkinson, la sclérose latérale amyotrophique, la névralgie faciale, l'atteinte du nerf glosso-pharyngien, la névralgie, la paralysie de Bell, la myasthénie grave, la dystrophie musculaire, l'atrophie musculaire progressive, l'atrophie musculaire bulbaire progressive génétique, les syndromes des disques intervertébraux herniés, rompus, ou en prolapsus, la spondylose cervicale, les troubles au niveau du plexus, les syndromes de destruction de l'orifice inférieur du thorax, le syndrome de Gullain-Barré, et les lésions du nerf sciatique.

11. Utilisation selon la revendication 10, dans laquelle le trouble neurologique est la maladie d'Alzheimer.

12. Utilisation selon la revendication 10, dans laquelle le trouble neurologique est la maladie de Parkinson.

13. Utilisation selon la revendication 10, dans laquelle le trouble neurologique est la sclérose latérale amyotrophique.

14. Utilisation d'un composé tel que défini dans la revendication 1 ou la revendication 2, pour la préparation d'un médicament pour traiter l'alopécie et/ou pour favoriser la croissance capillaire chez un animal.
